# EUROPÄISCHE PATENTSCHRIFT

(11) **EP 0 821 585 B1**
(45) Veröffentlichungstag und Bekanntmachung des Hinweises auf die Patenterteilung: **10.01.2007**
(21) Anmeldenummer: 96914912.9
(22) Anmeldetag: 13.04.1996
(51) Int. Cl.: A61K 31/4168, C07D 233/50, C07D 403/12, A61P 13/02

(54) **VERWENDUNG VON ALPHA 1L-AGONISTEN ZUR BEHANDLUNG DER HARNINKONTINENZ**
USE OF ALPHA 1L-AGONISTS IN THE TREATMENT OF URINARY INCONTINENCE
UTILISATION D'AGONISTES ALPHA 1L POUR LE TRAITEMENT DE L'INCONTINENCE URINAIRE

(30) Priorität: 20.04.1995 DE 19514579
(43) Veröffentlichungstag der Anmeldung: 04.02.1998
(60) Teilanmeldung: 02025309.2
(73) Patentinhaber: Boehringer Ingelheim Pharma GmbH & Co.KG, 55218 Ingelheim am Rhein (DE)
(72) Erfinder: ESSER, Franz, D-55218 Ingelheim (DE); STAEHLE, Helmut, D-55218 Ingelheim (DE); LUETTKE, Sven, D-55437 Ockenheim (DE); MURAMATSU, Ikunobu, Fukui 910-11 (JP); KITAGAWA, Hisato, Osaka Prefecture 563-01 (JP); UCHIDA, Shuji, M., D., Toyonaka 560 (JP)
(74) Vertreter: Kläs, Heinz-Gerd
(86) Internationale Anmeldenummer: PCT/EP1996/001568
(87) Internationale Veröffentlichungsnummer: WO 1996/032939

(56) Entgegenhaltungen:
- EP-A- 0 070 084
- EP-A- 0 081 923
- EP-A- 0 081 924
- EP-A- 0 117 102
- EP-A- 0 149 140
- EP-A- 0 236 636
- EP-A- 0 416 841
- EP-A- 0 464 946
- EP-A- 0 599 697
- EP-A- 0 682 028
- WO-A-94/08040
- DE-A- 2 806 811
- DE-A- 2 854 659
- US-A- 4 226 773
- US-A- 4 287 201
- US-A- 4 587 257
- US-A- 5 480 871
- PROG.CLIN.BIOL.RES., Bd. 78, 1981, Seiten 101-103, XP000602071 J.NORDLING ET AL.: "Sympathetic Influence on Striated Urethral Sphincter in Urinary Incontinence"
- JPN.J.PHARMACOL., Bd. 58, Nr. 4, 1992, Seiten 339-346, XP000602099 H.KONTANI ET AL.: "Effects of Adrenergic Agonists on an Experimental Urinary Incontinence Model in Anesthetized Rabbits"
- UROLOGY, Bd. 43, Nr. 3, März 1994, Seiten 324-327, XP000602098 J.G. VAN SAVAGE ET AL.: "EFFECTS OF ALPHA-ADRENERGIC AGONIST ON NEOBLADDER WATER AND ELECTROLYTE TRANSPORT"
- BIOCHEM. PHARMACOL., Bd. 32, Nr. 12, 1983, Seiten 1933-1940, XP000601225 W.C.RANDALL ET AL.: "MULTIPLE CENTRAL ALPHA2 ADRENOCEPTORS OF AVIAN AND MAMMALIAN SPECIES"

## Beschreibung

Die vorliegende Erfindung betrifft die Verwendung von selektiven α_{1L}-Agonisten für die Herstellung von Arzneimitteln zur Behandlung der Harninkontinenz, insbesondere der Streßinkontinenz.

Die Ursache der weiblichen Streßinkontinenz ist meist eine Beckenbodenschwäche, z.B. nach mehreren schweren Geburten. Sie kann aber auch auf Innervationsstörungen des Beckenbodens, einer angeborenen zu kurzen Harnröhre oder selten auf operativen Verletzungen des Schließmuskels beruhen. Die Abnahme der Östrogenspiegel in der Postmenopause fördert die Streßinkontinenz.

Als Streßinkontinenz wird plötzlicher Hamverlust bezeichnet, der durch eine Blasenauslaßinkompetenz bei unauffälliger Blasenmotorik während des Auftretens von interaabdominalen Drucksteigerungen durch Husten, Pressen, Niesen, schwerem Heben usw. verursacht ist.

Überraschenderweise hat sich gezeigt, daß der α_{1L}-Subtyp des adrenergen Rezeptors einen signifikanten Einfluß auf den Kontinenzmechanismus der Urethertonisierung hat.

Die Erfindung betrifft die Verwendung von selektiven α_{1L} -Adrenozeptoragonisten zur Behandlung der Harninkontinenz, insbesondere der Streßinkontinenz, beziehungsweise für die Herstellung von Arzneimitteln zur Behandlung der Harninkontinenz, insbesondere der Streßinkontinenz. Von besonderem Interesse ist die Verwendung von Aminoimidazolinen der allgemeinen Formel 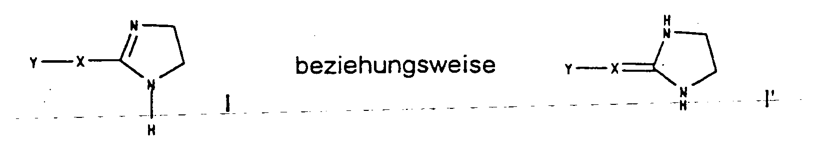 sowie deren pharmakologisch verträglichen Säureadditionssalzen. In der allgemeinen Formel I bedeuten
Y einen gegebenenfalls substituierter Phenyl- oder Naphthylrest und
X -NH.

Bevorzugt für diese Verwendung sind Imidazoline der allgemeinen Formel 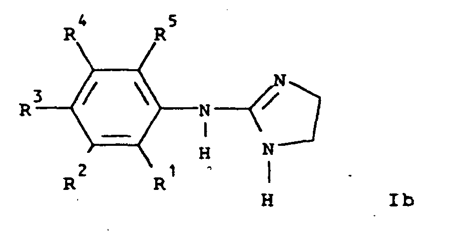 bzw. Imidazolidine der allgemeinen Formel 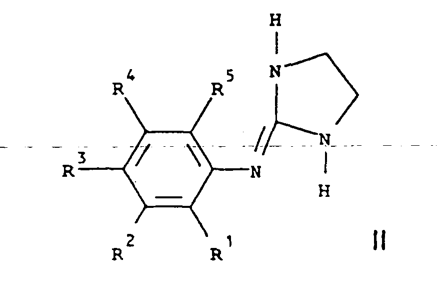 worin
R¹, R², R³, R⁴, R⁵ unabhängig voneinander wie folgt definiert sind:

Wasserstoff, C₁-C₆-Alkyl, bevorzugt C₁-C₄-Alkyl, besonders bevorzugt Methyl, C₃-C₆-Cycloalkyl, bevorzugt Cyclopropyl, C₁-C₆-Alkoxy, bevorzugt C₁-C₄-Alkoxy, besonders bevorzugt Methoxy, Halogen, bevorzugt Chlor oder Brom, CF₃, -OCF₃, oder NR⁶R⁷ mit
R6 Wasserstoff, C₃-C₆-Cycloalkyl, C₁-C₆-Alkyl, bevorzugt C₁-C₄-Alkyl, besonders bevorzugt Methyl, oder C₂-C₄-Acyl, besonders bevorzugt Acetyl,
R⁷ Wasserstoff, C₃-C₆-Cycloalkyl, bevorzugt Cyclopropyl, C₁-C₆-Alkyl, bevorzugt C₁-C₄-Alkyl, besonders bevorzugt Methyl, oder C₂-C₄-Acyl, besonders bevorzugt Acetyl;
oder
R⁶ und R⁷ bilden zusammen mit dem Stickstoffatom einen 5- oder 6-gliedrigen gesättigten oder ungesättigten Ring, der bis zu zwei weitere Heterotatome aus der Gruppe Sauerstoff, Schwefel oder Stickstoff enthalten kann, wobei jedes weitere Stickstoffatom durch C₁-C₄-Alkyl, bevorzugt Methyl, substituiert sein
kann;
oder R⁶ und R⁷ bilden zusammen mit dem Stickstoffatom
Phthalimido;
oder
R¹ und R² bilden zusammen ein ankondensiertes Pyrazol der Formel 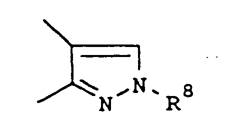 worin R⁸ C₁-C₃-Alkyl, bevorzugt Methyl ist, oder ein ankondensiertes Thiadiazol der Formel 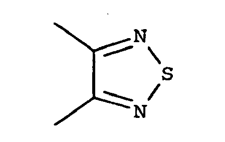 wobei R³, R⁴ und R⁵ wie zuvor definiert sind, und bevorzugt Wasserstoff bedeuten, sowie deren pharmakologisch verträgliche Säureadditionssalze.

Die Formel I und I' beziehungsweise Ib und II stellen gleichwertige tautomere Strukturen dar. Die Darstellung der einen Struktur (z.B. lb) schließt jeweils die andere Struktur (z.B. II) ein.

Bevorzugt ist ferner die Verwendung von lmidazolinen der allgemeinen Formel lb 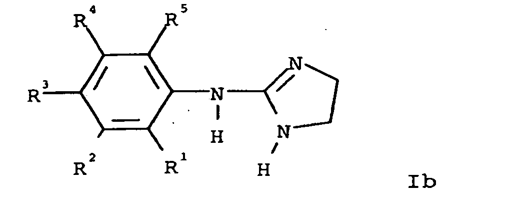 worin
- R¹: Wasserstoff, Ethyl, Methyl, Fluor, Chlor, Brom oder CF₃ ist,
- R²: Methyl, Fluor, Chlor, Brom oder -NR⁶R⁷ ist, worin
- R⁶: Wasserstoff, C₁-C₄-Alkyl, bevorzugt Methyl, C₂-C₄-Acyl, bevorzugt Acetyl und
- R⁷: Wasserstoff, C₁-C₄-Alkyl, bevorzugt Methyl, C₂-C₄-Acyl, bevorzugt Acetyl ist
oder
- R⁶: und R⁷ zusammen mit dem Stickstoffatom Phthalimido bilden;

- R³: Wasserstoff, Fluor, Chlor, Brom, C₁-C₄-Alkyl, bevorzugt Methyl, NH₂ oder Cyclopropyl ist;
- R4: Wasserstoff, C₁-C₄-Alkyl, bevorzugt Methyl, Fluor, Chlor, Brom oder CF₃ ist;
- R⁵: Wasserstoff, C₁-C₄-Alkyl, bevorzugt Ethyl oder Methyl, Fluor, Chlor, Brom oder CF₃ ist;
oder
- R¹: und R² zusammen ein ankondensiertes Pyrazol der Formel 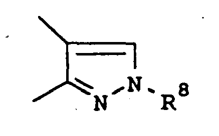 bilden, worin R⁸ Methyl ist,
oder ein ankondensiertes Thiadiazol der Formel 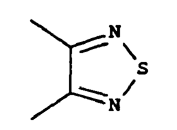 wobei R³, R⁴ und R⁵ wie zuvor definiert sind, und bevorzugt Wasserstoffbedeuten; insbesondere solche, worin
R¹ Wasserstoff oder Methyl ist;
R² Methyl, Chlor, CF₃, NH₂ oder N(CH₃)₂ ist;
R³ Wasserstoff, Methyl, Chlor oder Brom ist;
R⁴ Wasserstoff ist;
R⁵ Wasserstoff, Methyl, Methoxy, Chlor oder Brom ist.

Bevorzugt sind auch Verbindungen gemäß Anspruch 4.
Besonders hervorgehoben wird die Verwendung von
2-(3-Dimethylamino-2-methylphenylimino)imidazolidin,
2-(6-Brom-3-dimethylamino-2-methylphenylimino)imidazolidin,
2-(5-Amino-2-chlor-4-methylphenylimino)-imidazolidin,
2-(3-Amino-2-methylphenylimino)-imidazolidin oder
2-(2-Chlor-5-trifluormethylphenylimino)-imidazolidin.

Als heterocyclische Beispiele für den Rest NR⁶R⁷ werden genannt:

Pyrrol, Δ²-Pyrrolin, Δ³-Pyrrolin, Tetrahydropyrrol, Pyrrolidin, Pyrrolidinon, Imidazol, Imidazolin, 1,3-Thiazol, Piperidin, Piperazin, 4-C₁ bis C₄-Alkylpiperazin, C₁ bis C₄-Alkylpiperazin, 2,5-Diketopiperazin, bevorzugt N-Methylpiperazin, Morpholin, Thiomorpholin, Phthalimido, Succinimido.

Als Alkyl im Sinne der vorliegenden Definition werden - auch soweit sie Bestandteil anderer Reste sind - verzweigte oder unverzweigte Alkylgruppen mit 1 bis 6 Kohlenstoffatomen verstanden, beispielsweise werden genannt: Methyl, Ethyl, n-Propyl-, iso Propyl, iso-Butyl, n-Butyl, iso-Butyl, sec.-Butyl und tert.-Butyl, n-Pentyl, iso-Pentyl, neo-Pentyl, Hexyl, iso-Hexyl.

Cycloalkyl steht im allgemeinen für einen gesättigten cyclischen Kohlenwasserstoffrest mit 3 bis 6 Kohlenstoffatomen, der gegebenenfalls mit einem Halogenatom oder mehreren Halogenatomen, einer Hydroxygruppe, einer Alkylgruppe, bevorzugt Methyl substituiert sein kann, die untereinander gleich oder verschieden sein können. Als Beispiele seien Cyclopropyl, Cyclobutyl, Cyclopentyl, Cyclopentenyl, Cyclohexyl, Cyclohexenyl genannt.

Ein Teil der in der allgemeinen Formel lb definierten Imidazoline ist neu. Die Erfindung betrifft deshalb auch neue substituierte 2-Phenylimino-imidazolidine, gemäß Anspruch 9, ihre Verwendung als Arzneimittel sowie Verfahren in ihrer Herstellung.

2-(Phenylimino)-imidazolidine, ihre Herstellung und ihre Verwendung als Arzneimittel sind bekannt, so z.B. aus den DE-OS'en 19 29 950 und 23 16 377, wobei die blutdrucksenkende Eigenschaften der dort beschriebenen Verbindungen im Vordergrund stehen.

Besonders hervorzuheben sind beispielsweise
2-(3-Dimethylamino-2-methylphenylimino)imidazolidin, und
2-(6-Brom-3-dimethylamino-2-methylphenylimino)imidazolidin,

Die Verbindungen der allgemeinen Formel I und II können nach an sich bekannten Analogieverfahren aus dem Stand der Technik hergestellt werden. Eine Auswahl der bevorzugten Verfahren ist in den nachfolgenden Syntheseschemata anhand konkreter Beispiele beispielhaft dargestellt.

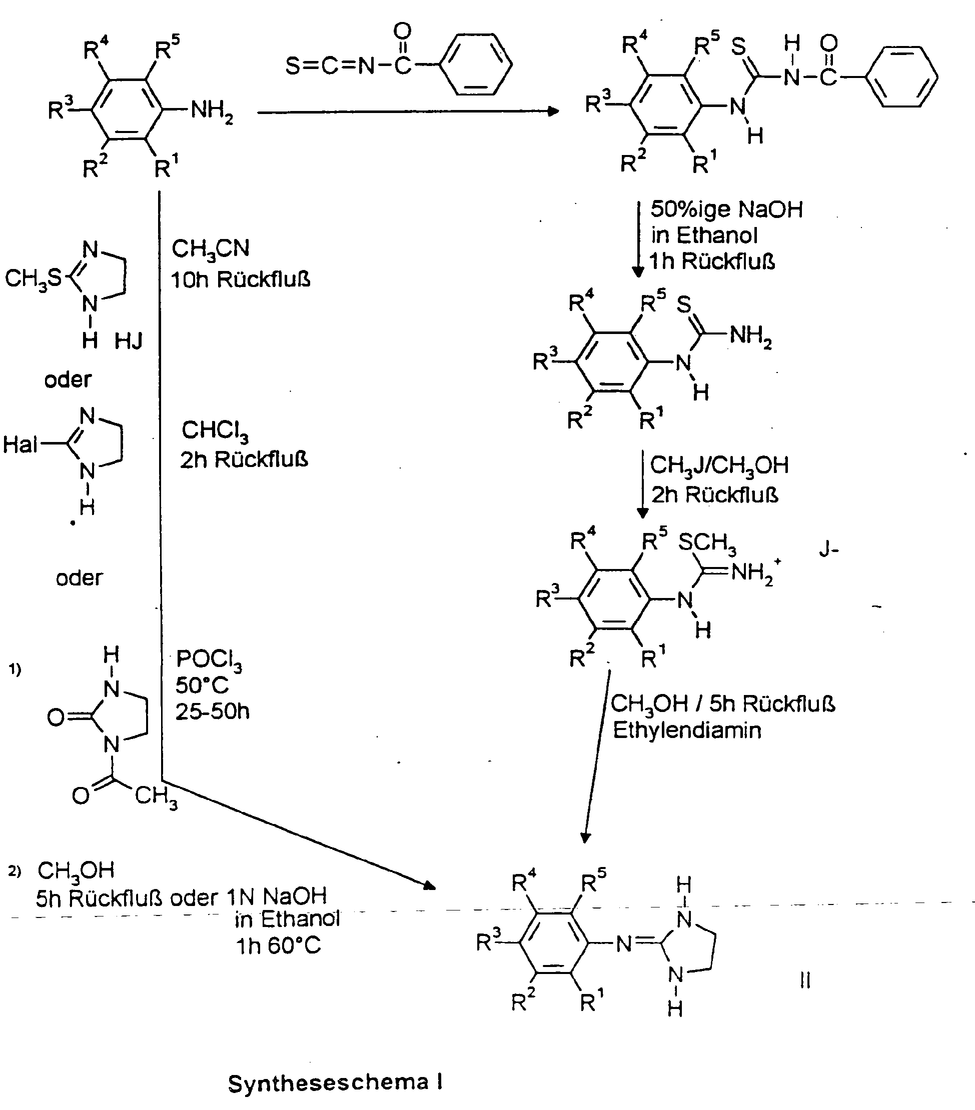

Anhand einzelner Beispiele werden die bevorzugten Verfahren zur Herstellung der erfindungsgemäßen Verbindungen erläutert.

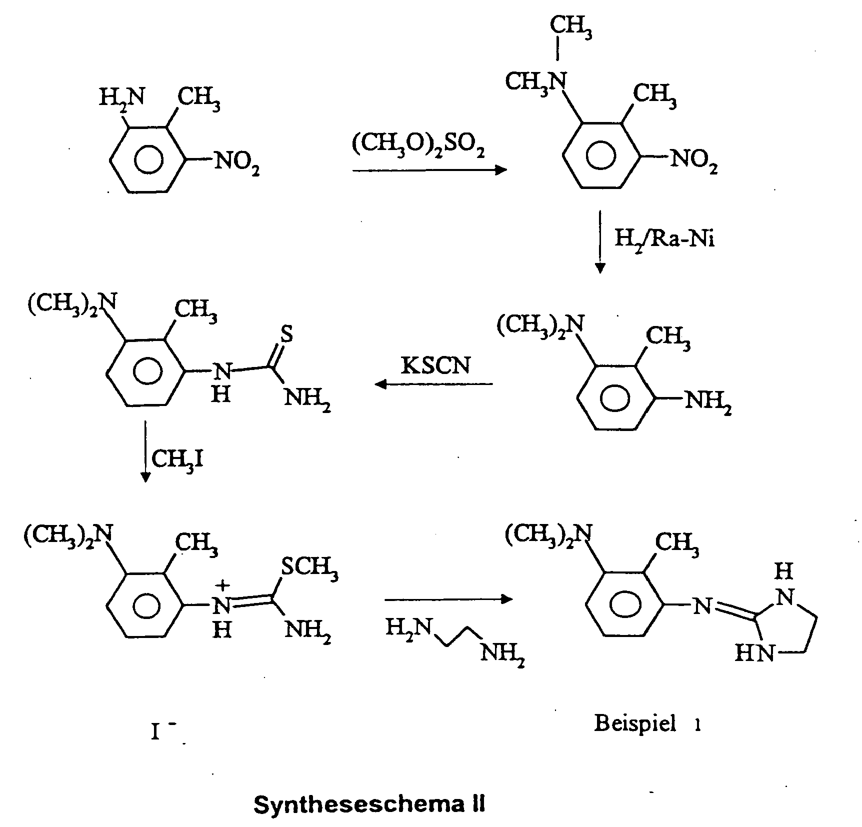

Die Methylierung des Ausgangsmaterials, des 2-Methyl-3-nitro-anilins kann auch in Analogie zur Leuckart-Wallach Reaktion unter Verwendung von HCOOH/CH₂O oder unter Verwendung von Dimethylcarbonat anstelle von Dimethylsulfat erfolgen.

Die Verbindung 2 ist durch Bromierung von Verbindung 1 unter üblichen Reaktionsbedingungen herstellbar

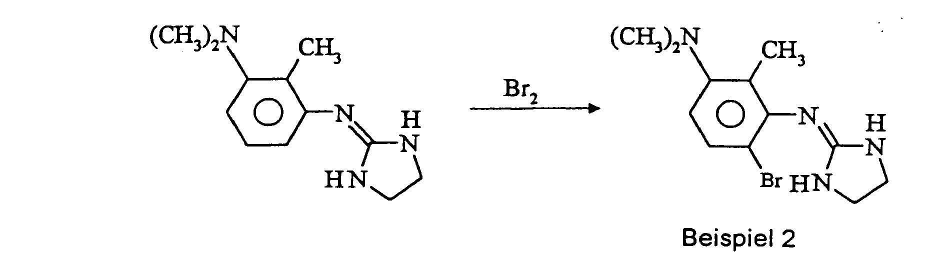

Das nachfolgende Syntheseschema erläutert die Herstellung der Verbindungen 2, 3 und 4

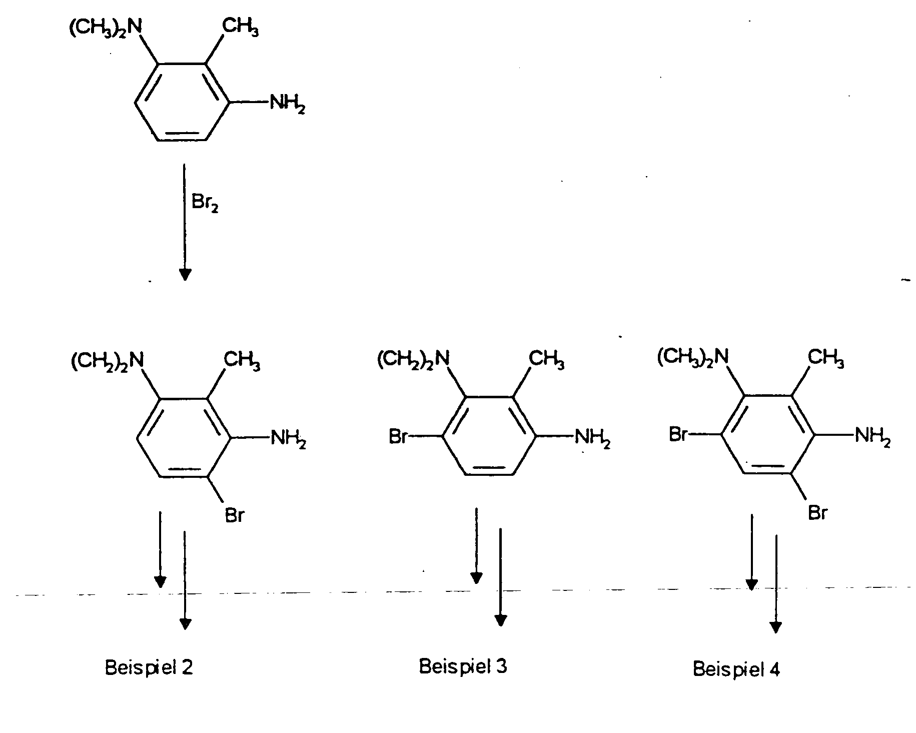

Weitere Synthesevarianten sind nachfolgend dargestellt.

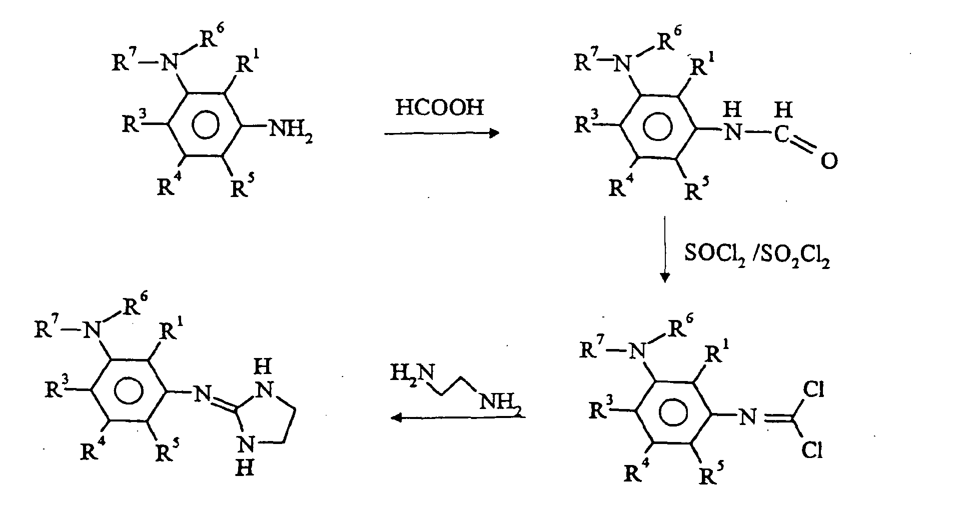

In Analogie zu einer von N.R. Ayyangar (Synthesis 1987, 64) beschriebenen Methode kann die Verbindung 5 und strukturähnliche Verbindungen hergestellt werden.

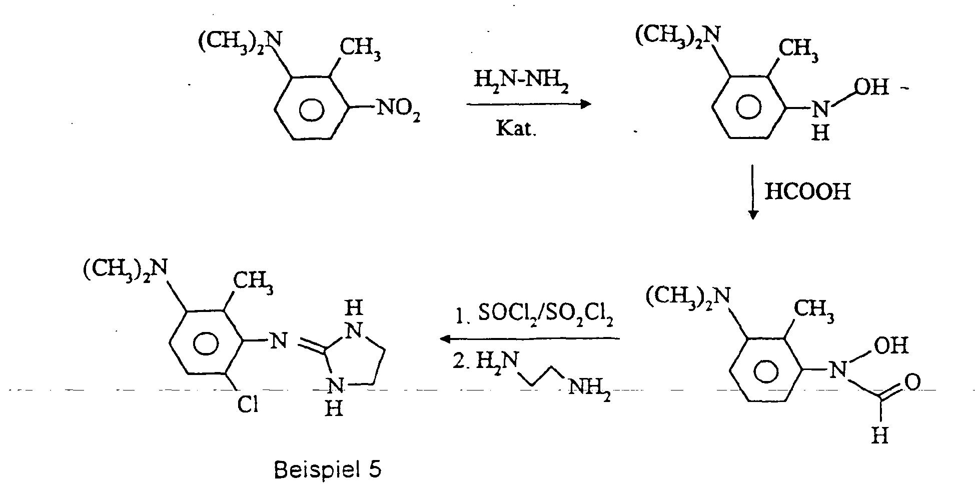

### Beispiel 1

### 2-(3-Dimethylamino-2-methylphenylimino)imidazolidin

### 1. Stufe:

83.6g 2-Methyl-3-nitroanilin, 190 g K₂CO₃ und 260 ml Wasser werden zusammen auf 100°C erhitzt. 27 ml Dimethylsulfat werden innerhalb 1 Stunde zugetropft, anschließend wird eine weitere Stunde erhitzt. Nach dem Erkalten auf Raumtemperatur wird die obere Schicht abgetrennt und die verbleibende wässerige Phase viermal mit Ether extrahiert.

Die vereinigten Etherextrakte werden mit der oberen Schicht vereint, mit MgSO₄ getrocknet, und im Vakuum eingeengt. Man erhält 73 g N,N-Dimethyl-2-methyl-3-nitroanilin.

### 2. Stufe:

73 g N,N-Dimethyl-2-methyl-3-nitroanilin werden in 800 ml Methanol gelöst und bei 20°C und 5 bar Wasserstoff unter Verwendung von Raney-Nickel als Katalysator hydriert. Man erhält 57 g 3-Dimethylamino-2-methylanilin.

### 3. Stufe:

57 g 3-Dimethylamino-2-methyl-anilin, 1,15 l Aceton, 36,6 g KSCN und 43.8 ml Benzoylchlorid werden zusammen 3 Stunden zum Rückfluß erhitzt. Nach dem Abkühlen auf Raumtemperatur wird das Reaktionsgemisch auf 2,4 kg zerstoßenes Eis gegeben. Der erhaltene Niederschlag wird zusammen mit 85 g KOH, 85 ml Wasser und 255 ml Ethanol für 2 Stunden auf 60°C erhitzt. Nach Zugabe von 850 ml Wasser wird unter vermindertem Druck das Ethanol abdestiliert. Man erhält nach der Aufarbeitung des resultierenden Niederschlages 72 g N-(3-Dimethylamino-2-methylphenyl)-thioharnstoff.

### 4. Stufe:

72 g des Thioharnstoffs aus Stufe 3 werden in 345 ml Methanol aufgenommen und nach Zugabe von 22,6 ml Methyljodid für 2 Stunden zum Rückfluß erhitzt. Die resultierende Lösung wird unter vermindertem Druck eingeengt; man erhält 120 g N-(3-Dimethylamino-2-methylphenyl)-S-methyl-isothioharnstoff Hydrojodid.

### 5. Stufe:

120 g des Thioharnstoffs aus Stufe 4 wird in 350 ml Methanol mit 34,4 ml 1,2-Diaminoethan 17 Stunden zum Rückfluß erhitzt. Das Reaktionsgemisch wird anschließend im Vakuum eingeengt und der Rückstand in Wasser aufgenommen. Mit verdünnter Salzsäure wird auf pH 7 eingestellt. Die wässerige Phase wird 3mal mit Ethylacetat extrahiert. Anschließend wird die wässerige Phase mit 5N NaOH alkalisch gestellt und noch 3mal mit Ethylacetat extrahiert, diese Extrakte werden vereinigt, mit MgSO₄ getrocknet und im Vakuum eingeengt. Man erhält ein Öl, das über Silicagel chromatographiert wird (Fließmittel Toluol, Dioxan, Ethanol, Ammoniak 10:8:3:1 = "Super-T').

Man erhält 17.9 g 2-(3-Dimethylamino-2-methylphenyl-imino)imidazolidine. Schmelzpunkt 116 - 118°C.

### Beispiel 2:

### 2-(6-Brom-3-dimethylamino-2-methylphenylimino)imidazolidin

6,55 g 2-(3-Dimethylamino-2-methylphenyl-imino)imidazolidin werden in 75 ml Chloroform gelöst und unter Rühren bei 0°C mit 1,53 ml Brom versetzt. Nach zwei Stunden bei 0°C wird die Lösung unter vermindertem Druck eingeengt und der so erhaltene Rückstand mit verdünnter Salzsäure versetzt. Die wässerige Lösung wird zweimal mit Ether extrahiert - anschließend wird die wässerige Phase mit verdünnter NaOH alkalisch gestellt und noch dreimal mit Ether extrahiert. Die vereinigten Etherextrakte werden unter vermindertem Druck eingeengt und der verbleibende Rückstand chromatographisch aufgearbeitet (Silicalgel, Fließmittel "Super-T" (Bsp. 1)).

Man erhält 3.4 g 2-(6-Brom-3-dimethylamino-2-methyl-phenylimino)-imidazolidin vom Schmp. 157 - 158°C als weißes Pulver.

In Analogie zu den beschriebenen Verfahren wurden folgende Verbindungen hergestellt:
2-(4-Brom-3-dimethylamino-2-methylphenylimino)-imidazolidin
2-(4,6-Dibrom-3-dimethylamino-2-methylphenylimino)-imidazolidin
2-(6-Chlor-3-dimethylamino-2-methylphenylimino)-imidazolidin
2-(3-Acetylamino-6-chlorphenylimino)-imidazoligin, Schmp. 236 -238°C
2-(2-Methyl-3-phtalimidophenylimino)-imidazolidin, Schmp. 189 - 190°C
2-(6-Chlor-3-phtalimidophenylimino)-imidazolidin, Schmp. 239 - 241 °C
2-(5-Amino-2-chlor-4-methylphenylimino)-imidazolidin, Schmp..155 - 157°C, auch bekannt aus DE 2806811
2-(3-Amino-4-fluorphenylimino)-imidazolidin, (2HCl), Schmp. 222°C, als Base bekannt aus DE 2854659
2-(3-Amino-4-methylphenylimino)-imidazolidin, (HCl),
2-(3-Amino-6-methylphenylimino)-imidazolidin, (HCl), Schmp. 194 - 196°C, als Dihydrochlorid bekannt aus EP 0 149 140
2-(3-Amino-6-chlorphenylimino)-imidazolidin, (HCl), Schmp. 197 - 198°C, auch bekannt aus EP 0 070 084 und EP 0 117 102
2-(3-Amino-4,6-dibrom-2-methylphenylimino)-imidazolidin, Schmp. 154 - 155°C
2-(3-Amino-2-methylphenylimino)-imidazolidin, (HCl), Schmp. 204 - 206°C

Im einzelnen werden folgende Verbindungen namentlich genannt:
2-(2,6-Diethylphenyl-imino)-imidazolidin
2-(2-Chlor-6-methylphenylimino)-imidazolidin
2-(2,6-Dichlor-phenylimino)-imidazolidin
2-(2-Chlor-4-methylphenylimino)-imidazolidin
2-(2,4-Dichlorphenylimino)-imidazolidin
2-(2-Chlor-5-trifluormethylphenylimino)-imidazolidin
2-(5-Fluor-2-methylphenylimino)-imidazolidin
2-(3-Brom-2-methylphenylimino)-imidazolidin
2-(2-Chlor-3-methylphenylimino)-imidazolidin
2-(2-Fluor-6-trifluormethylphenylimino)-imidazolidin
2-(2-Chlor-4-cyclopropylphenylimino)-imidazolidin
2-(4-Amino-3,5-dibromphenylimino)-imidazolidin
2-(3-Fluor-4-methylphenylimino)-imidazolidin
2-(6-Brom-2-fluorphenylimino)-imidazolidin
4-(2-Imidazolin-2-ylamino)-2-methylindazol
5-Chlor-4-(imidazolin 2-yl-amino)-benzothiadiazol(Tizanidine)
2-[(2-Chlor-4-methyl-3-thienyl)amino]-2-imidazolin (Tiamenidin)
2-(2,5-Dichlorphenylimino)-imidazolidin

Die erfindungsgemäßen Verbindungen der allgemeinen Formel I und II können auf übliche Weise in ihre physiologisch verträglichen Säureadditionssalze überführt werden. Zur Salzbildung geeignete Säuren sind beispielsweise Mineralsäuren, wie Salzsäure, Bromwasserstoffsäure, Jodwasserstoffsäure, Fluorwasserstoffsäure, Schwefelsäure, Phosphorsäure, Salpetersäure oder organische Säuren wie Essigsäure, Propionsäure, Buttersäure, Capronsäure, Caprinsäure, Valeriansäure, Oxalsäure, Malonsäure, Bernsteinsäure, Maleinsäure, Fumarsäure, Milchsäure, Weinsäure, Zitronensäure, Apfelsäure, Benzoesäure, p-Hydroxybenzoesäure, p-Aminobenzoesäure, Phthalsäure, Zimtsäure, Salicylsäure, Ascorbinsäure, Methansulfonsäure, Ethanphosphonsäure.

Bevorzugt sind die entsprechenden Hydrobromide und -chloride als Säureadditionssalze.

Pharmazeutische Zubereitungen, die die beschriebenen Verbindungen enthalten, können verwendet werden in Form von Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragées durch Überziehen von analog den Tabletten hergestellten Kernen mit üblicherweise in Dregéeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Dragéehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbessemdes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die den Wirkstoff, beziehungsweise die Wirkstoffkombination enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyethylenglykol, beziehungsweise dessen Derivate, herstellen.

Zum Zweck der transdermalen Applikation können die erfindungsgemäßen Wirkstoffe in entsprechend geeignete Träger (Pflaster), beispielsweise aus Polyacrylaten, eingearbeitet werden. Geeignete Adjuvantien können eingesetzt werden, um die Freigaberate zu erhöhen.

Als therapeutisch wirksame Einzeldosis wird bei oralen Applikationen eine Dosis von 1 bis 50 mg vorgeschlagen.

### Beispiel A: Tabletten

| | |
|---|---|
| 2-(3-Dimethylamino-2-methylphenylimino)-imidazolidin HBr | 10 mg |
| Michzucker | 65 mg |
| Maisstärke | 125 mg |
| sek. Calciumphosphat | 40 mg |
| lösliche Stärke | 3 mg |
| Magnesiumstearat | 4 mg |
| kolloidale Kieselsäure | 4 mg |
| insgesamt | 251 mg |

### Herstellung:

Der Wirkstoff wird mit einem Teil der Hilfsstoffe vermischt, intensiv mit einer wäßrigen Lösung der löslichen Stärke durchgeknetet und in üblicher Weise mit Hilfe eines Siebes granuliert. Das Granulat wird mit dem Rest der Hilfsstoffe vermischt und zu Dragéekernen von 250 mg Gewicht verpreßt, die dann in üblicher Weise mit Hilfe von Zucker, Talkum und Gummi arabicum dragiert werden.

### Beispiel B: Ampullen

| | |
|---|---|
| 2-(3-Dimethylamino-2-methylphenylimino)-imidazolidin HBr | 1,0 mg |
| Natriumchlorid | 18,0 mg |
| dest. Wasser ad | 2,0 ml |

### Herstellung:

Wirkstoff und Natriumchlorid werden in Wasser gelöst und unter Stickstoff in Glasampullen abgefüllt.

### Beispiel C: Tropfen

| | |
|---|---|
| 2-(3-Dimethylamino-2-methylphenylimino)-imidazolidin HBr | 0,02 g |
| p-Hydroxybenzoesäuremethylester | 0,07 g |
| p-Hydroxybenzoesäurepropylester | 0,03 g |
| entmineralisiertes Wasser ad | 100 ml |

### Beispiel D: Injektionslösung

| | |
|---|---|
| 2-(3-Dimethylamino-2-methylphenylimino)-imidazolidin HBr | 1,5 Teile |
| Natriumsalz der Ethylendiamintetraessigsäure | 0,2 Teile |
| dest. Wasser ad | 100,0 Teile |

### Herstellung:

Der Wirkstoff und das Natriumsalz der Ethylendiamintetraessigsäure werden in genügend Wasser gelöst und mit Wasser auf das gewünschte Volumen aufgefüllt. Die Lösung wird von suspendierten Partikeln filtriert und in 2-ml-Ampullen unter aseptischen Bedingungen abgefüllt. Zuletzt werden die Ampullen sterilisiert und verschlossen. Jede Ampulle enthält 20 mg Wirkstoff.

Ein Vorteil der beschriebenen Verbindungen beruht darauf, daß sie in erster Linie auf die Urethra wirken und geringe oder keine Wirkung auf das cardiovasculäre System zeigen.

Die selektive pharmakologische Wirkung der erfindungsgemäßen Verbindungen werden am Beispiel 2 - dem 2-(6-Brom-3-dimethylamino-2-methylphenylimino)-imidazolidin - und einer Vergleichsverbindung, dem Phenylephrin - durch Messung des intraluminalen Druckes der Urethra und des Blutdrucks beim Kaninchen gezeigt.

Weibliche japanische weiße Kaninchen (Gewicht 3,0 bis 3,5 kg) werden mit Urethan anesthesiert (1 g/kg i.p.). Eine Polyethylenkanüle wird mittels eines kleinen Schnittes in die Harnblase eingeführt. Die Änderungen des intraluminalen Druckes werden über einen Ballon in der Urethra bestimmt, der ca. 1,5 ml Wasser bei 37°C enthält. Der intraurethrale Druck wird mittels eines Druckspannungswandlers auf einen Polygraph aufgezeichnet.

Im geöffneten Halsbereich wird die Ateria carotis kanüliert um den Blutdruck zu messen, gleichzeitig wird zur Aufrechterhaltung der Atmung die Trachea intubiert.
Die Änderungen des Blutdruckes werden über einen Druckspannungswandler auf einen Polygraph aufgezeichnet. Die Herzfrequenz wurde unter Verwendung eines Tachometers gemessen.

Phenylephrin und die Verbindung des Beispiels 2 werden i.v. über eine Polyethylenkanüle in die Vena femoralis gegeben. Verglichen werden Dosierungen von 30 µg/kg Phenylephrin mit 10 µg/kg der Verbindung des Beispiels 2.

Verglichen mit Phenylephrin zeigt die Verbindung des erfindungsgemäßen Beispiels 2 hinsichtlich der Kontraktion der Urethra eine um den Faktor 2,73 höhere Wirkungsstärke sowie eine um den Faktor 4,3 längere Wirkungsdauer. Im Vergleich dazu beträgt die Erhöhung des Blutdruckes bei der erfindungsgemäßen Verbindung lediglich das 1,39-fache gegenüber der Vergleichsverbindung Phenylephrin. Bemerkenswert ist, daß die Erhöhung des Blutdruckes im Vergleich zu Phenylephrin nur unwesentlich (Faktor 1,17) verlängert wird. Diese Versuche belegen, daß die erfindungsgemäßen Verbindungen selektiv auf die Urethra wirken. Als selektive α _{1L}-adrenorezeptor Agonisten sind die erfindungsgemäßen Verbindungen zur Behandlung von Haminkontinenzleiden, insbesondere zur Behandlung der Streßinkontinenz geeignet.

Die Versuchsergebnisse sind in Tabelle I zusammengestellt.

**Tabelle I**

| | Kontraktion der Urethra | Wirkungsdauer | Blutdruckerhöhung | Wirkungsdauer - |
|---|---|---|---|---|
| Phenylephrin | 100 | 100 | 100 | 100 |
| Beispiel 2 | 273 | 430 | 139 | 117 |

Angaben in %
Beispiel 2 = 2-(6-Brom-3-dimethylamino-2-methylphenyl-imino)imidazolidin

## Patentansprüche

1. Verwendung von selektiven α_{1L}-Agonisten der allgemeinen Formel I für die Herstellung von Arzneimitteln zur Behandlung der Harninkontinenz, insbesondere der Streßhaminkontinenz 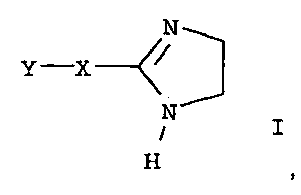 worin
Y ein gegebenenfalls substituierter Phenyl- oder Naphthylrest ist und
X -NH- bedeutet,
sowie deren pharmakologisch verträgliche Säureadditionssalze.

2. Verwendung nach Anspruch 1, wobei die selektiven α_{1L}-Agonisten der allgemeinen Formel lb 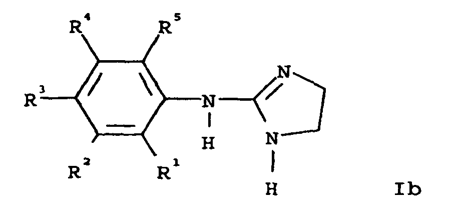 entsprechen,
worin R¹, R², R³, R⁴, R⁵ unabhängig voneinander wie folgt definiert sind:
Wasserstoff, C₁-C₆-Alkyl, C₃-C₆-Cycloalkyl, C₁-C₆-Alkoxy, Halogen, CF₃, -OCF₃ oder NR⁶R⁷ mit
R⁶ Wasserstoff, C₃-C₆-Cycloalkyl, C₁-C₆-Alkyl, oder C₂-C₄-Acyl,
R⁷ Wasserstoff, C₃-C₆-Cycloalkyl, C₁-C₆-Alkyl, oder C₂-C₄-Acyl; oder
R⁶ und R⁷ bilden zusammen mit dem Stickstoffatom einen 5- oder 6-gliedrigen gesättigten oder ungesättigten Ring, der bis zu zwei weitere Heterotatome aus der Gruppe Sauerstoff, Schwefel oder Stickstoff enthalten kann, wobei jedes weitere Stickstoffatom durch C₁-C₄-Alkyl substituiert sein kann;
oder R⁶ und R⁷ bilden zusammen mit dem Stickstoffatom Phthalimido;
oder
R¹ und R² bilden zusammen ein ankondensiertes Pyrazol der Formel 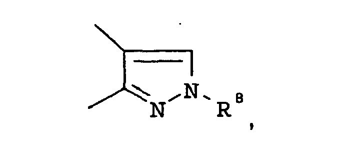 worin R⁸ C₁-C₃-Alkyl ist,
oder ein ankondensiertes Thiadiazol der Formel 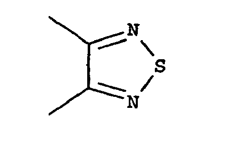 wobei R³, R⁴ und R⁵ wie zuvor definiert sind, sowie deren pharmakologisch verträglichen Säureadditionssalzen zur Herstellung von Arzneimitteln zur Behandlung der Harninkontinenz, insbesondere der Streßinkontinenz.

3. Verwendung nach Anspruch 2,
worin R¹, R², R³, R⁴, R⁵ unabhängig voneinander wie folgt definiert sind:
Wasserstoff, C₁-C₄-Alkyl, bevorzugt Methyl, Cyclopropyl, C₁-C₄-Alkoxy, bevorzugt Methoxy, Halogen, CF₃, -OCF₃ oder NR⁶R⁷ mit
R⁶ Wasserstoff, C₃-C₆-Cycloalkyl, C₁-C₄-Alkyl, bevorzugt Methyl, oder Acetyl,
R⁷ Wasserstoff, Cyclopropyl, C₁-C₄-Alkyl, bevorzugt Methyl, oder Acetyl;
oder
R⁶ und R⁷ bilden zusammen mit dem Stickstoffatom Phthalimido;
oder
R¹ und R² bilden zusammen ein ankondensiertes Pyrazol der Formel 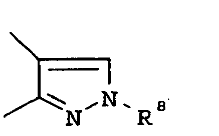 worin R⁸ Methyl ist,
oder ein ankondensiertes Thiadiazol der Formel 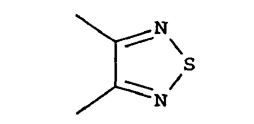 wobei R³, R⁴ und R⁵ wie zuvor definiert sind, und bevorzugt Wasserstoff bedeuten.

4. Verwendung nach Anspruch 2,
worin R¹, R², R³, R⁴, R⁵ unabhängig voneinander wie folgt definiert sind:
Wasserstoff, Ethyl, Methyl, Cyclopropyl, Fluor, Chlor, Brom, CF₃ oder NR⁶R⁷ mit
R⁶ Wasserstoff, Methyl oder Acetyl,
R⁷ Wasserstoff, Methyl oder Acetyl;
oder
R⁶ und R⁷ bilden zusammen mit dem Stickstoffatom Phthalimido; oder
R¹ und R² bilden zusammen ein ankondensiertes Pyrazol der Formel 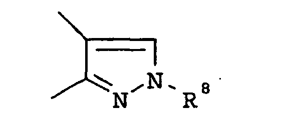 worin R⁸ Methyl ist,
oder ein ankondensiertes Thiadiazol der Formel 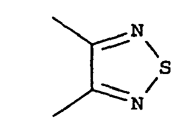 wobei R³, R⁴ und R⁵ wie zuvor definiert sind, und bevorzugt Wasserstoff bedeuten.

5. Verwendung von Phenylaminoimidazolinen der allgemeinen Formel lb nach Anspruch 2 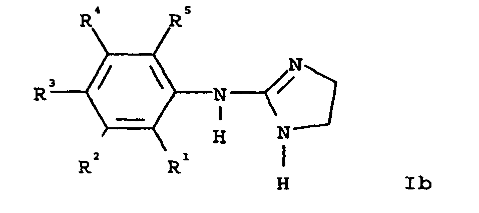 worin
R¹ Wasserstoff, Ethyl, Methyl, Fluor, Chlor, Brom oder CF₃ ist,
R² Methyl, Fluor, Chlor, Brom oder -NR⁶R⁷ ist, worin
R⁶ Wasserstoff, C₁-C₄-Alkyl, bevorzugt Methyl, C₂-C₄-Acyl, bevorzugt Acetyl und
R⁷ Wasserstoff, C₁-C₄-Alkyl, bevorzugt Methyl, C₂-C₄-Acyl, bevorzugt Acetyl ist
oder
R⁶ und R⁷ zusammen mit dem Stickstoffatom Phthalimido bilden;
R³ Wasserstoff, Fluor, Chlor, Brom, C₁-C₄-Alkyl, bevorzugt Methyl, NH₂ oder Cyclopropyl ist;
R⁴ Wasserstoff, C₁-C₄-Alkyl, bevorzugt Methyl, Fluor, Chlor, Brom oder CF₃ ist;
R⁵ Wasserstoff, C₁-C₄-Alkyl, bevorzugt Ethyl oder Methyl, Fluor, Chlor, Brom oder CF₃ ist;
oder
R¹ und R² zusammen ein ankondensiertes Pyrazol der Formel 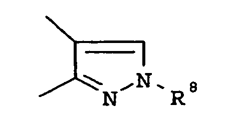 bilden, worin R⁸ Methyl ist,
oder ein ankondensiertes Thiadiazol der Formel 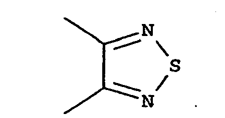 wobei R³, R⁴ und R⁵ wie zuvor definiert sind, und bevorzugt Wasserstoff bedeuten.

6. Verwendung nach Anspruch 3, worin
R¹ Wasserstoff oder Methyl ist;
R² Methyl, Chlor, CF₃, NH₂ oder N(CH₃)₂ ist;
R³ Wasserstoff, Methyl, Chlor oder Brom ist;
R⁴ Wasserstoff ist;
R⁵ Wasserstoff, Methyl, Methoxy, Chlor oder Brom ist.

7. Verwendung nach Anspruch 6, worin die Verbindung
2-(3-Dimethylamino-2-methylphenylimino)imidazolidin,
2-(6-Brom-3-dimethylamino-2-methylphenylimino)imidazolidin,
2-(5-Amino-2-chlor-4-methylphenylimino)-imidazolidin,
2-(3-Amino-2-methylphenylimino)-imidazolidin
oder
2-(2-Chlor-5-trifluormethylphenylimino)-imidazolidin ist.

8. Phenyliminoimidazolidin, das
2-(2-Methyl-3-phtalimidophenylimino)-imidazolidin;
2-(4,6-Dibrom-3-dimethylamino-2-methylphenylimino)-imidazolidin;
2-(4-Brom-3-dimethylamino-2-methylphenylimino)-imidazolidin;
2-(6-Brom-3-dimethylamino-2-methylphenylimino)imidazolidin;
2-(6-Chlor-3-dimethylamino-2-methylphenylimino)-imidazolidin;
2-(6-Chlor-3-phtalimidophenylimino)-imidazolidin;
2-(3-Dimethylamino-2-methylphenylimino)imidazolidin,
2-(6-Brom-3-dimethylamino-2-methylphenylimino)imidazolidin ist.

9. Pharmazeutische Zubereitung enthaltend eine Verbindung nach Anspruch 8 sowie übliche Hilfs- und/oder Trägerstoffe.

10. Verfahren zur Herstellung von pharmazeutischen Zubereitungen gemäß Anspruch 9, **dadurch gekennzeichnet, daß** man Verbindungen nach Anspruch 8 mit üblichen galenischen Hilfs- und/oder Trägerstoffen mischt.

11. Verwendung von Verbindungen nach Anspruch 8 für die Herstellung von Arzneimitteln zur Behandlung der Harninkontinenz, insbesondere Streßinkontinenz.

## Claims

1. Use of selective α_{1L}-agonists of general formula I for preparing pharmaceutical compositions for the treatment of urinary incontinence, particularly stress incontinence, 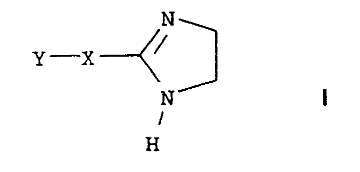 wherein
Y denotes an optionally substituted phenyl or napthyl group and
X denotes -NH-,
and the pharmacologically acceptable acid addition salts thereof.

2. Use according to claim 1, wherein the selective α_{1L}-agonists correspond to general formula Ib 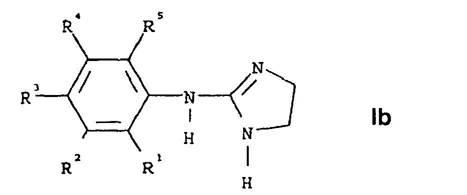 wherein
R¹, R², R³, R⁴ and R⁵ denote, independently of one another:
hydrogen, C₁₋₆-alkyl, C₃₋₆-cycloalkyl, C₁₋₆-alkoxy, halogen, CF₃, -OCF₃ or NR⁶R⁷, wherein
R⁶ denotes hydrogen, C₃₋₆-cycloalkyl, C₁₋₆-alkyl, or C₂₋₄-acyl,
R⁷ denotes hydrogen, C₃₋₆-cycloalkyl, C₁₋₆-alkyl, or C₂₋₄-acyl;
or
R⁶ and R⁷ together with the nitrogen atom form a 5- or 6-membered saturated or unsaturated ring which may contain up to two further heteroatoms selected from oxygen, sulphur or nitrogen, whilst each additional nitrogen atom may be substituted by C₁₋₄-alkyl;
or R⁶ and R⁷ together with a nitrogen atom form phthalimido;
or
R¹ and R² together form a fused-on pyrazole of formula 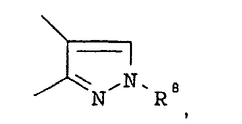 wherein R⁸ is C₁₋₃-alkyl,
or a fused-on thiadiazole of formula 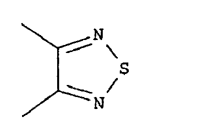 wherein R³, R⁴ and R⁵ are as hereinbefore defined, and the pharmacologically acceptable acid addition salts thereof, for preparing pharmaceutical compositions for treating urinary incontinence, particularly stress incontinence.

3. Use according to claim 2,
wherein R¹, R², R³, R⁴, R⁵ denote independently of one another:
hydrogen, C₁₋₄-alkyl, preferably methyl, cyclopropyl, C₁₋₄-alkoxy, preferably methoxy, halogen, CF₃, -OCF₃ or NR⁶R⁷, wherein
R⁶ denotes hydrogen, C₃₋₆-cycloalkyl, C₁₋₄-alkyl, preferably methyl, or acetyl,
R⁷ denotes hydrogen, cyclopropyl, C₁₋₄-alkyl, preferably methyl, or acetyl;
or
R⁶ and R⁷ together with the nitrogen atom form phthalimido; or
R¹ and R² together form a fused-on pyrazole of formula 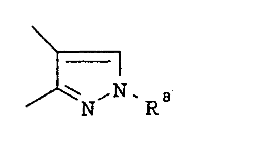 wherein R⁸ is methyl,
or a fused-on thiadazole of the formula 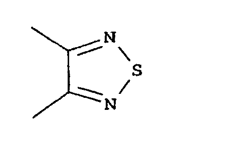 wherein R³, R⁴ and R⁵ are as hereinbefore defined, and preferably denote hydrogen.

4. Use according to claim 2,
wherein R¹, R², R³, R⁴, R⁵ denote independently of one another:
hydrogen, ethyl, methyl, cyclopropyl, fluorine, chlorine, bromine, CF₃ or NR⁶R⁷ wherein
R⁶ denotes hydrogen, methyl or acetyl,
R⁷ denotes hydrogen, methyl or acetyl;
or
R⁶ and R⁷ together with the nitrogen atom form phthalimido; or
R¹ and R² together form a fused-on pyrazole of the formula 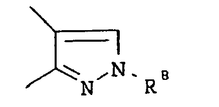 wherein R⁸ is methyl,
or a fused-on thiadiazole of the formula 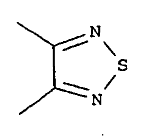 wherein R³, R⁴ and R⁵ are as hereinbefore defined, and preferably represent hydrogen.

5. Use of phenylaminoimidazolines of general formula 1 b according to claim 2 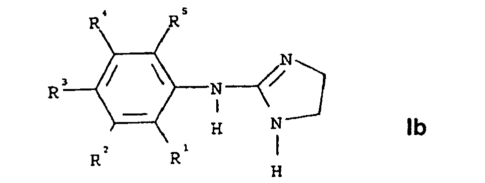 wherein
R¹ is hydrogen, ethyl, methyl, fluorine, chlorine, bromine or CF₃,
R² is methyl, fluorine, chlorine, bromine or -NR⁶R⁷, wherein
R⁶ is hydrogen, C₁₋₄-alkyl, preferably methyl, C₂₋₄-acyl, preferably acetyl, and
R⁷ is hydrogen, C₁₋₄-alkyl, preferably methyl, C₂₋₄-acyl, preferably acetyl
or
R⁶ and R⁷ together with the nitrogen atom form phthalimido;
R³ is hydrogen, fluorine, chlorine, bromine, C₁₋₄-alkyl, preferably methyl, NH₂ or cyclopropyl;
R⁴ is hydrogen, C₁₋₄-alkyl, preferably methyl, fluorine, chlorine, bromine or CF₃;
R⁵ is hydrogen, C₁₋₄-alkyl, preferably ethyl or methyl, fluorine, chlorine, bromine or CF₃;
or
R¹ and R² together form a fused-on pyrazole of the formula 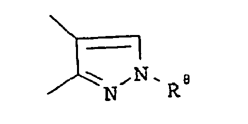 wherein R⁸ is methyl,
or a fused-on thiadiazole of the formula 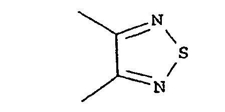 wherein R³, R⁴ and R⁵ are as hereinbefore defined, and preferably denote hydrogen.

6. Use according to claim 3, wherein,
R¹ is hydrogen or methyl;
R² is methyl, chlorine, CF₃, NH₂ or N(CH₃)₂;
R³ is hydrogen, methyl, chlorine or bromine;
R⁴ is hydrogen;
R⁵ is hydrogen, methyl, methoxy, chlorine or bromine.

7. Use according to claim 6, wherein the compound is
2-(3-dimethylamino-2-methylphenylimino)imidazolidine,
2-(6-bromo-3-dimethylamino-2-methylphenylimino)imidazolidine,
2-(5-amino-2-chloro-4-methylphenylimino)-imidazolidine,
2-(3-amino-2-methylphenylimino)-imidazolidine
or
2-(2-chloro-5-trifluoromethylphenylimino)-imidazolidine.

8. Phenyliminoimidazolidine, which is
2-(2-methyl-3-phthalimidophenylimino)-imidazolidine;
2-(4,6-dibromo-3-dimethylamino-2-methylphenylimino)-imidazolidine;
2-(4-bromo-3-dimethylamino-2-methylphenylimino)-imidazolidine;
2-(6-bromo-3-dimethylamino-2-methylphenylimino)imidazolidine;
2-(6-chloro-3-dimethylamino-2-methylphenylimino)-imidazolidine;
2-(6-chloro-3-phthalimidophenylimino)-imidazolidine;
2-(3-dimethylamino-2-methylphenylimino)imidazolidine;
2-(6-bromo-3-dimethylamino-2-methylphenylimino)imidazolidine.

9. Pharmaceutical preparation containing a compound according to claim 8 together with conventional excipients and/or carriers.

10. Process for producing pharmaceutical preparations according to claim 9. **characterised in that** compounds according to claim 8 are mixed with conventional galenic excipients and/or carriers.

11. Use of compounds according to claim 8 for preparing pharmaceutical compositions for the treatment of urinary incontinence, particularly stress incontinence.

## Revendications

**1.** Utilisation d'α_{1L}-agonistes sélectifs de formule générale I pour la production de médicaments pour le traitement de l'incontinence urinaire, en particulier de l'incontinence urinaire à l'effort 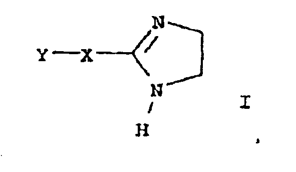 où
Y est un groupement phényle ou naphtyle éventuellement substitué et
X représente -NH-,
ainsi que de leurs sels d'addition d'acide pharmacologiquement acceptables.

**2.** Utilisation selon la revendication 1 où les α_{1L}-agonistes sélectifs correspondent à la formule générale Ib 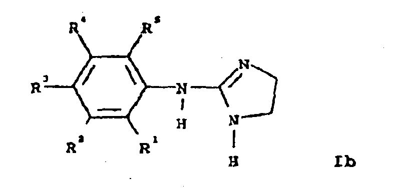 où R¹, R², R³, R⁴, R⁵ sont définis indépendamment les uns des autres comme suit : l'hydrogène, C₁-C₆-alkyle, C₃-C₆-cycloalkyle, C₁-C₆-alcoxy, halogène, CF₃, - OCF₃ ou NR⁶R⁷ avec
K⁶ l'hydrogène, C₃-C₆-cycloalkyle, C₁-C₆-alkyle ou C₂-C₄-acyle,
R⁷ l'hydrogène, C₃-C₆-cycloalkyle, C₁-C₆-alkyle ou C₂-C₄-acyle ;
ou
R⁶ et R⁷ forment avec l'atome d'azote un cycle saturé ou insaturé à 5 ou 6 chaînons qui peut contenir jusqu'à deux autres hétéroatomes du groupe oxygène, soufre ou azote, où chaque autre atome d'azote peut être substitué par C₁-C₄-alkyle ;
ou R⁶ et R⁷ forment avec l'atome d'azote phtalimido ;
ou
R¹ et R² forment ensemble un pyrazole condensé de formule 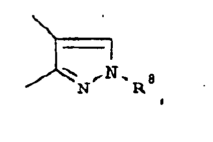 où R⁸ est C₁-C₃-alkyle,
ou un thiadiazole condensé de formule 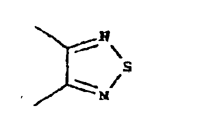 où R³, R⁴ et R⁵ sont définis comme précédemment, ainsi que leurs sels d'addition d'acide pharmacologiquement acceptables pour la production de médicaments pour le traitement de l'incontinence urinaire, en particulier de l'incontinence à l'effort.

**3.** Utilisation selon la revendication 2
où R¹, R², R³, R⁴, R⁵ sont définis indépendamment les uns des autres comme suit : l'hydrogène, C₁-C₄-alkyle, de préférence méthyle, cyclopropyle, C₁-C₄-alcoxy, de préférence méthoxy, halogène, CF₃, -OCF₃ ou NR⁶R⁷ avec
R⁶ l'hydrogène, C₃-C₆-cycloalkyle, C₁-C₄-alkyle, de préférence méthyle, ou acétyle, R⁷ l'hydrogène, cyclopropyle, C₁-C₄-alkyle, de préférence méthyle, ou acétyle ;
ou
R⁶ et R⁷ forment avec l'atome d'azote phtalimido ;
ou
R¹ et R² forment ensemble un pyrazole condensé de formule 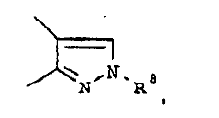 où R⁸ est méthyle,
ou un thiadiazole condensé de formule 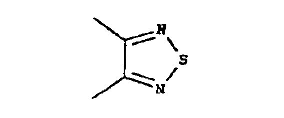 où R³, R⁴ et R⁵ sont définis comme précédemment, et représentent de préférence l'hydrogène.

**4.** Utilisation selon la revendication 2
où R¹, R², R³, R⁴, R⁵ sont définis indépendamment les uns des autres comme suit : l'hydrogène, éthyle, méthyle, cyclopropyle, fluor, chlore, brome, CF₃ ou NR⁶R⁷ avec
R⁶ l'hydrogène, méthyle ou acétyle,
R⁷ l'hydrogène, méthyle ou acétyle ;
ou
R⁶ et R⁷ forment avec l'atome d'azote phtalimido ;
ou
R¹ et R² forment ensemble un pyrazole condensé de formule 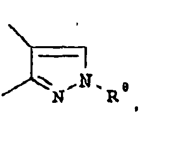 où R⁸ est méthyle,
où un thiadiazole condensé de formule 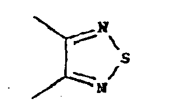 où R³, R⁴ et R⁵ sont définis comme précédemment, et représentent de préférence l'hydrogène.

**5.** Utilisation de phénylaminoimidazolines de formule générale Ib selon la revendication 2 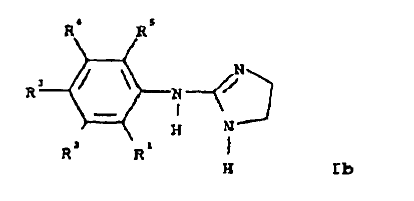 où
R¹ est l'hydrogène, éthyle, méthyle, le fluor, le chlore, le brome ou CF₃,
la 2-(3-diméthylamino-2-méthylphénylimino)imidazolidine,
la 2-(6-bromo-3-diméthylamino-2-méthylphénylimino)imidazolidine,
la 2-(5-amino-2-chloro-4-méthylphénylimino)imidazolidine,
la 2-(3-amino-2-méthylphénylimino)imidazolidine
ou
la 2-(2-chloro-5-trifluorométhylphénylimino)imidazolidine.

**8.** Phényliminoimidazolidine qui est
la 2-(2-méthyl-3-phtalimidophénylimino)imidazolidine ;
la 2-(4,6-dibromo-3-diméthylamino-2-méthylphénylimino)imidazolidine ;
la 2-(4-bromo-3-diméthylamino-2-méthylphénylimino)imidazolidine ;
la 2-(6-bromo-3-diméthylamino-2-méthylphénylimino)imidazolidine ;
la 2-(6-chloro-3-diméthylamino-2-méthylphénylimino)imidazolidine ;
la 2-(6-chloro-3-phtalimidophénylimino)imidazolidine ;
la 2-(3-diméthylamino-2-méthylphénylimino)imidazolidine ;
la 2-(6-bromo-3-diméthylamino-2-méthylphénylimino)imidazolidine.

**9.** Préparation pharmaceutique contenant un composé selon la revendication 8 ainsi que des adjuvants et/ou supports habituels.

**10.** Procédé de production de préparations pharmaceutiques selon la revendication 9 **caractérisé en ce que** l'on mélange des composés selon la revendication 8 avec des adjuvants et/ou supports galéniques habituels.

**11.** Utilisation de composés selon la revendication 8 pour la production de médicaments pour le traitement de l'incontinence urinaire, en particulier de l'incontinence à l'effort.
